Europäisches Patentamt

European Patent Office

Office européen des brevets

(19)

(11) Numéro de publication: **0 284 492**
**A1**

(12) **DEMANDE DE BREVET EUROPEEN**

(21) Numéro de dépôt: 88400609.9

(22) Date de dépôt: 15.03.88

(51) Int. Cl.4: **A 61 K 39/21**
C 07 K 7/00, C 07 K 1/12,
C 07 K 17/00, A 61 K 39/42,
C 12 N 15/00

(30) Priorité: 20.03.87 FR 8703881

(43) Date de publication de la demande:
28.09.88 Bulletin 88/39

(84) Etats contractants désignés:
AT BE CH DE ES FR GB GR IT LI NL SE

(71) Demandeur: RHôNE MERIEUX
17, rue Bourgelat
F-69002 Lyon (FR)

(72) Inventeur: Portetelle, Daniel Georges Joseph Ghislain
11 rue de la Motte
B-5854 Meux-la-Bruyère (BE)

Burny, Arsène Léon Ghislain
65 chaussée de Namur
B-5800 Gembloux (BE)

Dandoy, Corine Francine
8 rue des Déportés
B-7490 Braine-le-Comte (BE)

Gras (née Masse), Hélène Suzanne Camille
La Rosière - Merignies
F-59710 Pont-à-Marcq (FR)

Tartar, André Léon
rue du Moulin
F-62490 Vitry-en-Artois (FR)

(74) Mandataire: Bernasconi, Jean et al
CABINET LEMOINE ET BERNASCONI 13, Boulevard des
Batignolles
F-75008 Paris (FR)

(54) Nouvelles fractions peptidiques inductrices d'anticorps protecteurs contre le virus de la leucémie bovine, procédé pour l'obtention de telles fractions, leurs séquences codantes et vaccins réalisés à partir de ces fractions.

(57) Fraction peptidique induisant la formation d'anticorps protecteurs contre le virus de la leucémie bovine (BLV), caractérisée en ce qu'elle comporte une séquence peptidique reproduisant tout ou partie de la séquence du fragment de l'enveloppe glycoprotéinique gp51 du virus de la leucémie bovine portant au moins l'un des épitopes (F, G, H,) responsables de l'activité biologique du virus. Cette fraction peut être le fragment lui-même ou un peptide de synthèse. Application à la préparation ou à la recherche d'anticorps, au diagnostic et à la préparation de vaccins.

Fig. 6

EP 0 284 492 A1

**0 284 492**

## Description

Nouvelles fractions peptidiques inductrices d'anticorps protecteurs contre le virus de la leucémie bovine, procédé pour l'obtention de telles fractions, leurs séquences codantes et vaccins réalisés à partir de ces fractions.

La présente invention a trait à de nouvelles fractions peptidiques inductrices d'anticorps protecteurs contre le virus de la leucémie bovine (BLV) ainsi qu'à un procédé pour l'obtention de telles fractions.

Elle concerne également des vaccins réalisés à partir de ces fractions peptidiques ou de peptides de synthèse ayant en commun avec lesdites fractions au moins un site antigénique responsable de l'activité biologique du virus.

La leucémie bovine ou leucose bovine enzootique est une maladie hautement contagieuse induite par un rétrovirus, le virus de la leucémie bovine (BLV). Cette maladie, qui sévit principalement dans les pays de l'Europe de l'Est et en Amérique du Nord et du Sud, touch le bétail, essentiellement les ovins et les bovins, conduisant à une infection quasi généralisée des troupeaux. Il s'agit d'une maladie dont l'évolution est relativement lente, mais qui, dans de nombreux cas, induit des tumeurs, entraînant la mort de l'animal infecté.

Le développement de cette maladie est d'autant plus préoccupant que l'on ne dispose à l'heure actuelle pratiquement d'aucun moyen pour la combattre et pour éviter la propagation du virus. La solution consistait jusqu'ici à isoler les animaux infectés et à les abattre.

On a cherché depuis de nombreuses années à mettre au point un vaccin qui puisse apporter une certaine protection contre la leucémie bovine.

J. MILLER et M. VAN DER MAATEN ont mentionné dans Ann. Recherche Vét., p. 871 à 877, 9 (1978) la possibilité d'utiliser des glycoprotéines de l'enveloppe du virus BLV inactivé, comme principe actif d'un vaccin.

L.V. PATRASCU et al on décrit dans Rev. Méd. - Virologie, p. 955 à 1002, 31 (1980), la préparation d'un vaccin dénommé BL - VACC - RO contre le virus de la leucémie bovine, obtenu à partir d'un virus BLV inactivé.

M. MAMMERICKX, D. PORTETELLE, A. BURNY et J. LEUNEN rapportent dans Zbl. Vet. Med. B27, p. 291 à 303 (1981), les résultats d'études qui montrent que des anticorps passifs acquis par le colostrum protègent les animaux contre une infection.

M. ONUNA et al signalent dans Am. J. Vet. Res. 45, p. 1212 à 1215 (1984) que les anticorps contre la glycoprotéine d'enveloppe de poids moléculaire 51 000 du virus BLV désignée par l'abréviation gp51 montrent une activité neutralisante contre le virus BLV. Il est dans cet article fait état de vaccins réalisés à partir de la glycoprotéine gp51, de la protéine p24 et de cellules de reins d'agneau foetal (FLK) infectées et qui, administrés au mouton, apportent une certaine protection.

PARFANOVICH et al décrivent dans Br. Vet. J. 139, p. 137 à 146 (1983) la préparation d'un virus BLV inactivé à l'aide de composés aminométhylés provenant de la réaction entre la formaldéhyde et un amino-acide tel que la leucine ou la lysine.

G.H. THEILEN et al dans Current Topics in Veterinary Medicine and Animal Science 15, p. 547 à 559 (1982) indiquent avoir vacciné des bovins avec des cellules vivantes provenant d'une lignée cellulaire BL-3, obtenue à partir de moelle osseuse et de thymus d'un cas sporadique de leucose bovine.

Tous ces vaccins ne confèrent toutefois qu'une protection de très courte durée et aucun n'a reçu une application à grande échelle.

Il est connu que, dans certaines conditions, la glycoprotéine gp51 induit des anticorps neutralisants.

C. BRUCK et al. dans Virology 122, 342-352 (1982) ont mis en évidence huit régions antigéniques indépendantes sur la glycoprotéine gp51 par compétition d'anticorps monoclonaux anti-BLV.

D. PORTETELLE et al. dans Comm Eur. Communities (REP) EUR (1984), EUR 8471, Agriculture, 45-51 précisent que trois de ces épitopes, F, G, H, situés sur un fragment apparemment non glycosylé, de poids moléculaires d'environ 15.000, obtenu par digestion par une solution d'urokinase de la glycoprotéine gp51, sont impliqués dans la neutralisation du virus, et envisagent la possibilité de préparer un vaccin anti-BLV par insertion du gène d'enveloppe gp51 ou d'une région correspondant aux sites biologiquement actifs dans un système d'expression autorisant la glycosylation. Ils envisagent également la possibilité théorique de reproduire des épitopes efficaces sous forme de peptides de synthèse.

D. PORTETELLE et al., dans un abrégé paru dans J. Cell. Biochem. Supl. 10 A (1986), estiment que les trois épitopes F, G, H, peuvent jouer un rôle important dans la conception d'un vaccin de sous-unité anti-BLV. Ces trois épitopes sont sensibles à la présence d'un agent de réduction, sont localisés sur un fragment faiblement glycosylé sur la partie NH2 terminale de la glycoprotéine et sont les seuls épitopes reconnus sur le virion non dégradé. Une conférence correspondant à cet abrégé n'a pas été tenue.

Un des buts de la présente invention est, à partir de ces derniers travaux, de développer des fractions peptidiques susceptibles d'être incorporées dans un vaccin efficace.

Un des buts de la présente invention est de proposer un fragment de gp51 portant au moins l'un des épitopes responsables de l'activité biologique du virus et qui, en particulier, induisent la formation d'anticorps neutralisants, ledit fragment pouvant être utilisé comme principe actif dans un vaccin contre la leucémie bovine.

Un autre but de l'invention est de fournir des peptides de synthèse reproduisant au moins l'un des épitopes responsables de l'activité neutralisante, caractéristiques du fragment de gp51 et capables d'induire des anticorps qui reconnaissent, d'une part, les peptides à partir desquels ils ont été induits et, d'autre part, le virus BLV.

Un autre but encore de l'invention est de fournir des peptides de synthèse qui, éventuellement couplés avec un support protéinique ou autre approprié, sont susceptibles d'induire des anticorps qui neutralisent l'activité biologique du virus BLV.

Un autre but encore de l'invention est de fournir des peptides de synthèse pouvant servir de réactifs pour détecter la présence du virus BLV.

Un autre but encore de l'invention est de fournir un vaccin contre la leucémie bovine réalisé, soit à partir du fragment de glycoprotéine gp51 précité, soit à partir des peptides de synthèse, éventuellement couplés avec un support.

Conformément à l'invention, les sites antigéniques ou épitopes de la glycoprotéine gp51 sont mis en évidence par des anticorps monoclonaux dirigés contre la molécule de gp51.

Ces anticorps monoclonaux sont obtenus par "la technique de fabrication des hybridomes", bien connue en soi, consistant à fusionner des cellules myélomateuses et des cellules spléniques de souris ayant été au préalable immunisées avec la glycoprotéine gp51 puis à sélectionner parmi les hybridomes formés ceux qui sécrètent des anticorps monoclonaux actifs contre la glycoprotéine gp51.

Les anticorps monoclonaux permettent de mettre en évidence, notamment par les techniques immunoenzymatiques (par exemple méthode ELISA) ou radioimmunologiques, huit épitopes sur la molécule de gp51, désignés par les lettres A à H.

Afin de localiser sur la molécule de gp51 les sites antigéniques induisant la formation d'anticorps neutralisants, on a soumis la glycoprotéine gp51 à une digestion protéolytique ménagée, par exemple par l'urokinase, ce qui conduit à l'obtention de deux fragments : un premier fragment (fragment I) dont le poids moléculaire est d'environ 35 000 et un second fragment constitué par la partie NH$_2$ terminale (les 160 premiers acides aminés) faiblement glycolysée de poids moléculaire d'environ 15 000 (fragment II).

Une immunoprécipitation des fragments peptidiques avec des anticorps monoclonaux dirigés contre chacun des épitopes A à H, suivie d'une analyse par électrophorèse sur gel de polyacrylamide, montre que les trois épitopes F, G et H qui induisent la formation d'anticorps neutralisants, sont localisés sur le fragment II tandis que les épitopes A à D sont situés sur le fragment I de poids moléculaire d'environ 35 000.

La fraction peptidique induisant la formation d'anticorps protecteurs contre le virus de la leucémie bovine (BLV) conforme à l'invention est caractérisée en ce qu'elle comporte une séquence peptidique reproduisant tout ou partie de la séquence du fragment de l'enveloppe glycoprotéinique gp51 de virus BLV portant au moins l'un des épitopes responsables de l'activité neutralisante du virus.

Suivant l'un des modes de réalisation de l'invention, la fraction peptidique est constituée par le fragment de glycoprotéine gp51 lui-même, ce fragment se caractérisant :

- en ce qu'il représente la partie NH$_2$ terminale de la glycoprotéine gp51 ;
- en ce qu'il présente un poids moléculaire de l'ordre de 15 000 et en ce qu'il est faiblement glycosylé ;
- en ce qu'il porte dans une position accessible les épitopes induisant la formation d'anticorps neutralisants.

Ces épitopes sont eux-mêmes caractérisés :

- en ce qu'ils sont reconnus par les anticorps monoclonaux dirigés contre eux-mêmes ;
- en ce qu'ils sont reconnus de façon sélective par les sérums d'ovins ou de bovins infectés ;
- en ce qu'ils sont sensibles à une dénaturation du fragment par un agent réducteur.

L'activité neutralisante des anticorps induits par les épitopes F, G et H a été déterminée par le test d'inhibition des pseudotypes décrit par ZAVADA J., CERNY L., ZADADOVA A., BOZONOVA J. et ALTSTEIN A.D. dans le J. Natl. Cancer Inst. 62, 95-101 (1979).

La fraction peptidique conforme à l'invention est obtenue en soumettant la glycoprotéine gp51 à une digestion protéolytique ménagée, par exemple par une solution d'urokinase.

Les fragments peptidiques obtenus sont immunoprécipités par les anticorps monoclonaux. Les complexes protéines-anticorps formés sont ensuite séparés par électrophorèse sur gel de polyacrylamide en milieu dodécylsulfate de sodium, puis dissociés pour obtenir la fraction recherchée.

Partant de ces résultats, les inventeurs ont procédé à la sélection des peptides en vue de la synthèse chimique, en utilisant des procédés basés sur la prédiction du caractère hydrophile de séquences peptidiques comme par exemple le procédé décrit par KYTE et DOOLITTLE dans le Journal of Molecular Biology 157, 105 à 123 (1982) ou en utilisant des procédés basés sur la prédiction de la flexibilité de chaînes peptidiques comme par exemple le procédé décrit par KARPLUS et SCHULZ dans Naturwissenschaften 72, 212 à 215 (1982).

Les peptides de synthèse conformes à l'invention comportent une séquence peptidique cherchant à reproduire au moins l'un des épitopes F, G et H, caractéristiques du fragment de gp51 de poids moléculaire de l'ordre de 15 000.

Les peptides préférés sont constitués par ou comportent la formule ci-après :

Glu-Pro-Arg-Cys-Pro-Tyr-Val-Gly-Ala-Asp-His-Phe-Asp-Cys-Pro

laquelle formule correspond à la séquence 78-92 de la gp51.

Le polypeptide peut être linéaire ou cyclique, la cyclisation s'effectuant par la liaison S-S entre les 2 cystéines.

D'autres peptides intéressants peuvent notamment comprendre les séquences suivantes ou en être constitués :

```
Pro-Asp-Pro-Pro-Gln-Pro-Asp-Phe-Pro-Gln-Leu-Asn

Pro-Asp-Pro-Pro-Gln-Pro-Asp-Phe-Pro- Gln-Leu-Asn-Ser-Asp

Cys-Pro-Arg-Ser-Pro-Arg-Tyr-Thr-Asp-Leu

Cys-Ala-Lys-Ser-Pro-Arg-Tyr-Thr-Leu-Asp,
```

ces formules correspondant respectivement aux séquences 144-155, 144-157 et 39-48 de la glycoprotéine gp51.

Il va de soi que les fonctions réactives libres que sont susceptibles de posséder les différents résidus aminoacides entrant dans la constitution du peptide selon l'invention, peuvent être modifées, dès lors que cette modification n'entraîne pas une modification des propriétés immunogènes de l'ensemble du peptide. Les peptides ainsi modifiés entrent naturellement dans le cadre de la présente invention. C'est ainsi que des groupes -SH des résidus cystéine peuvent être à l'état thiol libre, disulfure, (par exemple dans le cas de peptides dimérisés ou cycliques) protégés par un groupe de protection, par exemple acétamidométhyle. De même les fonctions carboxyle peuvent éventuellement être acylées ou estérifiées et les groupes amines éventuellement alcoylés.

Les peptides selon l'invention peuvent être préparés par les techniques classiques, dans le domaine de la synthèse des peptides. Cette synthèse peut être réalisée en solution homogène ou en phase solide.

Par exemple on aura recours à la technique de synthèse en solution homogène décrite par HOUDENWEYL dans l'ouvrage intitulé "Methoden der Organischen Chemie" (Méthode de la chimie organique) édité par E. Wünsch., vol. 15-I et II, THIEME, STUTTGART 1974.

Cette méthode de synthèse consiste à condenser successivement deux à deux les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classiques, du type carbodiimide, tels que par exemple la 1-éthyl-3-(3-diméthylaminopropyl)-carbodiimide. Lorsque l'aminoacyle mis en oeuvre possède une fonction amine supplémentaire (cas de la lysine par exemple), ou une autre fonction acide (cas de l'acide glutamique par exemple), ces fonctions seront par exemple protégées, par des groupes carbobenzoxy ou t-butyloxycarbonyle, en ce qui concerne les fonctions amine, ou par des groupes t-butylester, en ce qui concerne les fonctions carboxyliques. Il en ira de même de la protection de toute autre fonction réactive. Par exemple, lorsque l'un des aminoacyles considérés contient une fonction S (par exemple la cystéine), on pourra avoir recours à un groupe acétamidométhyle ou paraméthoxybenzyle.

Dans le cas de la synthèse progressive, acide aminé par acide aminé, la synthèse débute de préférence par la condensation de l'amino-acide C-terminal avec l'amino-acide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 85, 2149-2154 (1963)).

Pour fabriquer une chaîne peptidique selon le procédé de MERRIFIELD, on a recours à une résine polymère très poreuse, sur laquelle on fixe le premier amino-acide C-terminal de la chaîne. Cet amino-acide est fixé sur la résine par l'intermédiaire de son groupe carboxylique et sa fonction amine est protégée, par exemple par le groupe t-butyloxycarbonyle.

Lorsque le premier amino-acide C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième amino-acide qui fournit le second aminoacyle de la séquence recherchée, à partir du résidu aminoacyle C-terminal sur la fonction amine déprotégée du premier amino-acide C-terminal fixé sur la chaîne. De préférence, la fonction carboxyle de ce deuxième amino-acide est activée, par exemple par le dicyclohexylcarbodiimide, et la fonction amine est protégée, par exemple par le t-butyloxycarbonyle.

On obtient ainsi la première partie de la chaîne peptidique recherchée, qui comporte deux amino-acides et dont la fonction amine terminale est protégée. Comme précédemment, on déprotège la fonction amine et on peut ensuite procéder à la fixation du troisième aminoacyle, dans des conditions analogues à celles de l'addition du deuxième amino-acide C-terminal.

On fixe ainsi, les uns après les autres, les acides aminés, qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents amino-acides constituant la chaîne peptidique et on détache le peptide de la résine, par exemple à l'aide d'acide fluorhydrique.

On peut également synthétiser, ou préparer d'une autre manière, une séquence d'ADN codant pour un peptide selon l'invention et l'introduire, en association avec un promoteur usuel, dans un vecteur d'expression tel que bactérie, levure, lignée cellulaire.

L'invention a également pour objet les séquences codantes d'ADN prévues à cet effet et les gènes purifiés constitués par ou comprenant ces séquences.

Chaque séquence d'ADN peut être déterminée à partir de la séquence d'acides du peptide selon l'invention par une transposition de routine.

Enfin, on peut également envisager de produire des peptides ou polypeptides selon l'invention par clivages sélectifs de la glycoprotéine gp51 ou de sa fraction II.

L'invention concerne également les oligomères hydrosolubles des peptides monomères sus-indiqués. L'oligomérisation peut entraîner un accroissement de l'immunogénicité des peptides selon l'invention. Sans qu'une telle indication chiffrée puisse être considérée comme limitative, on mentionnera néanmoins que ces oligomères peuvent éventuellement contenir de 2 à 10 unités monomères.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode préférée de l'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine ou d'autres groupes réactifs, par exemple -SH, en présence d'agents de couplage homo- ou hétéro-bifonctionnels.

L'invention concerne encore les conjugués obtenus par couplage covalent des peptides selon l'invention ou des susdits oligomères à des molécules poreuses qui peuvent être des protéines naturelles ou les protéines virales elles-mêmes ou encore des supports synthétiques, physiologiquement acceptables et non toxiques, par l'intermédiaire de groupements réactifs complémentaires respectivement portés par la molécule porteuse et le peptide. Des exemples de groupements appropriés sont illustrés dans ce qui suit.

A titre d'exemples de molécules porteuses ou supports macromoléculaires entrant dans la constitution des conjugués selon l'invention, on mentionnera des protéines naturelles, telles que l'anatoxine tétanique, l'ovalbumine, des sérums albumines, le keyhole Limpet Hemocyanin, la thyroglobuline, etc.

A titre de supports macromoléculaires synthétiques, on mentionnera par exemple des polylysines ou des poly(D-L-alanine)-poly(L-Lysine).

La littérature mentionne d'autres types de supports macromoléculaires susceptibles d'être utilisés, lesquels présentent en général un poids moléculaire supérieur à 20.000.

Pour synthétiser les conjugués selon l'invention, on peut avoir recours à des procédés connus en soi, tels que celui décrit par FRANTZ et ROBERTSON dans Infect. and Immunity, 33, 193-198 (1981), ou celui décrit dans Applied and Environmental Microbiology, octobre 1981, vol. 42, n° 4, 611-614 par P.E. KAUFFMAN en utilisant le peptide et la molécule porteuse appropriée.

Dans la pratique, on utilisera avantageusement comme agent de couplage les composés suivants, cités à titre non limitatif : aldéhyde glutarique, chloroformiate d'éthyle, carbodiimides hydrosolubles [N-éthyl-N′ (3-diméthylamino-propyl)carbodiimide, HCl], diisocyanates, bis-diazobenzidine, di- et trichloro-s-triazines, bromures de cyanogène, benzaquinone, ainsi que les agents de couplage mentionnés dans Scand. J. Immunol., 1978, vol. 8, p. 7-23 (AVRAMEAS, TERNYNCK, GUEDSON).

On peut avoir recours à tout procédé de couplage faisant intervenir, d'une part, une ou plusieurs fonctions réactives du peptide et, d'autre part, une ou plusieurs fonctions réactives des molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple, le 1-éthyl-3-(3-diméthylaminopropyl)-carbodiimide, le N-hydroxybenzotriazole, etc. On peut encore avoir recours à la glutaraldéhyde, notamment lorsqu'il s'agit de relier entre eux des groupes aminés respectivement portés par le peptide et la molécule support.

Il est également possible d'associer deux ou plusieurs peptides de synthèse conformes à l'invention par association covalente ou non, entre eux ou sur une protéine porteuse quelconque.

Les oligopeptides de synthèse, lorsqu'ils sont administrés à l'animal, provoquent chez celui-ci des anticorps capables de reconnaître dans le virus la séquence d'acides aminés correspondant aux constituants de synthèse.

Les peptides conformes à l'invention peuvent naturellement être préparés par d'autres moyens que la synthèse chimique, par exemple en utilisant des bactéries transformées par des vecteurs porteurs d'une séquence de nucléotides correspondant aux acides aminés.

Il va également de soi qu'entrent dans le cadre de la présente invention, les peptides dont certaines séquences d'acides aminés seraient modifiées, sans que soient modifiées sensiblement les propriétés immunologiques des peptides en cause.

La présente invention a également pour objet des vaccins obtenus soit à partir du fragment de glycoprotéine gp51 de poids moléculaire de l'ordre de 15 000, soit à partir des peptides de synthèse reproduisant au moins l'un des épitopes induisant la formation d'anticorps neutralisants, caractéristiques dudit fragment de gp51, et plus particulièrement du peptide 78-92, couplés ou non à un support.

Les compositions vaccinales qui associent la fraction peptidique ou les peptides de synthèse avec des véhicules pharmaceutiques et/ou des agents adjuvants, se présentent, en général, sous la forme de solutions injectables. De préférence, les doses unitaires seront faibles et ne dépasseront pas 1 µg/kg de poids vif.

Les adjuvants utilisés sont, soit des adjuvants huileux et émulsionnés avec l'antigène, soit de type adsorbant tel qu'hydroxyde d'aluminium.

D'autres caractéristiques et avantages de l'invention apparaîtront à la lecture du complément de description qui suit, se référant aux dessins annexés dans lesquels :

- la figure 1 illustre la mise en évidence par un test ELISA des huit épitopes de la glycoprotéine gp51 à l'aide d'anticorps monoclonaux ;

- la figure 2 illustre les résultats obtenus dans des tests de fixation lors desquels les anticorps monoclonaux spécifiques des huit épitopes de gp51 ont été mis en présence de quantités croissantes d'un sérum bovin infecté ;

- la figure 3 illustre les résultats d'une analyse par Western Blot de particules virales BLV ;

- la figure 4 représente, de façon schématique, la molécule de gp51 et l'endroit où s'effectue le clivage ;

- la figure 5 illustre la réactivité de différents anticorps monoclonaux avec différentes préparations de gp51 ;

- la figure 6 représente les séquences d'acides aminés de la glycoprotéine gp51 établies à partir de la séquence ADN de quatre variants de BLV ;

- la figure 7 représente les courbes de titration des sérums antipeptides de lapin contre la glycoprotéine gp51 ; et

- la figure 8 représente les courbes de titration des sérums antipeptides de lapin contre le virus BLV complet.

## PREPARATION DES ANTICORPS MONOCLONAUX

### IMMUNISATION DES SOURIS

On a injecté à des souris Balb/c par voie sous-cutanée et intrapéritonéale 50 µg de gp51 en présence d'adjuvant complet de Freund. Cette injection a été répétée 2 semaines après en présence d'adjuvant incomplet de Freund, puis 4 semaines après sans adjuvant. On a procédé, deux mois après, à une dernière injection par voie intrapéritonéale et par voie intraveineuse.

### FUSION DES CELLULES

On a préparé les hybrides par fusion de cellules de myélome de souris avec les splénocytes des souris immunisées selon la technique décrite par HERZENBERG L.A. et al. dans Handbook of Experimental Immunology (D. WEIR, ed.) 25.1-25 (Blackwell, London).

Les hybrides obtenus ont été répartis en plaques de 96 puits et sélectionnés sur milieu HAT en présence de thymocytes et de macrophages de souris. Les hybrides producteurs d'anticorps anti-gp51 ont été détectés à l'aide d'un radioimmunoessai en phase liquide avec l'antigène gp51 marqué à l'iode 125 ou à l'aide d'un radioimmunoessai en phase solide avec du virus BLV adsorbé dans les puits des microplaques. Dans ce dernier cas, les anticorps spécifiques adsorbés sur le virus ont été détectés avec un antiimmunoglobuline de souris marqué à l'iode 125. Après lavage, la radioactivité adsorbée sur les complexes spécifiques est révélée par autoradiographie.

Les clones producteurs sélectionnés ont été transférés sur des plaques de 24 puits et sous-clonés en milieu semi-solide d'agarose.

Les cellules hybrides obtenues ont été injectées dans la cavité intrapéritonéale de souris préalablement traitées au pristane. Après 10 à 15 jours, on a récolté les ascites qui contenaient des quantités importantes des anticorps monoclonaux recherchés.

On a purifié ces anticorps monoclonaux par chromatographie d'échange d'ions sur DEAE-Affigel blue selon la méthode décrite par Bruck et al. dans J. Immunological Methods 53, 313-319 (1982).

Ces anticorps monoclonaux ont été marqués par l'iode 125 par le procédé à la chloramine T décrit par GREENWOOD F.C. et al. dans Biochemical Journal, 89, 114-123 (1963).

La spécificité des différents anticorps monoclonaux obtenus pour des épitopes donnés a été déterminée par des essais de compétition entre les anticorps pour de l'antigène gp51 adsorbé dans les puits des plaques de microtitration.

A cet effet, un microgramme d'anticorps monoclonal purifié non marqué radioactivement est incubé, pendant une nuit à 4°C, dans un volume de 50µl dans les puits d'une plaque de microtitration en matière plastique contenant la gp51 adsorbée (50 ng).

De l'anticorps marqué radioactivement à l'iode 125 (10 ng-100.000 cpm) est alors ajouté et l'incubation se poursuit 6 heures de plus à 4°C.

Les microplaques sont alors lavées intensivement et la radioactivité accrochée à la matière plastique de chaque puits est mesurée dans un compteur.

On interprète les résultats en sachant que, si deux sites antigéniques sont très proches ou identiques, la liaison de l'un des anticorps (non marqué) à son épitope, va gêner ou empêcher la liaison du second anticorps (marqué) à son épitope.

Les essais de compétition entre les anticorps monoclonaux purifiés permettent de définir sur la molécule de gp51 huit sites antigéniques indépendants désignés par les lettres A, B, C, D, E, F, G, H.

## METHODE ELISA PERMETTANT DE DETECTER DES VARIANTS DU VIRUS BLV

Cette méthode a été mise en oeuvre dans les conditions qui suivent :

- fixation de 300 ng d'anticorps monoclonal purifié dirigé contre un site antigénique donné sur les parois des puits d'une plaque de microtitration,

- après lavage et saturation avec une protéine inerte, l'albumine bovine, introduction (en présence de la protéine inerte et de détergent Tween 80) de dilutions des protéines virales obtenues à partir de différents isolats de virus BLV ; incubation pendant 16 h à 4°C,

- après lavage, fixation sur l'antigène gp51 d'anticorps monoclonaux dirigés contre les sites antigéniques et couplés à l'enzyme peroxydase ; incubation pendant 4 h à 4°C,

- après lavage, révélation de l'activité enzymatique associée aux complexes à l'aide du substrat $H_2O_2$ + O-phénylène diamine,

- après 15 mn, arrêt de la réaction avec HCl 6N et lecture des densités optiques à l'aide d'un spectrophotomètre pour plaques de microtitration.

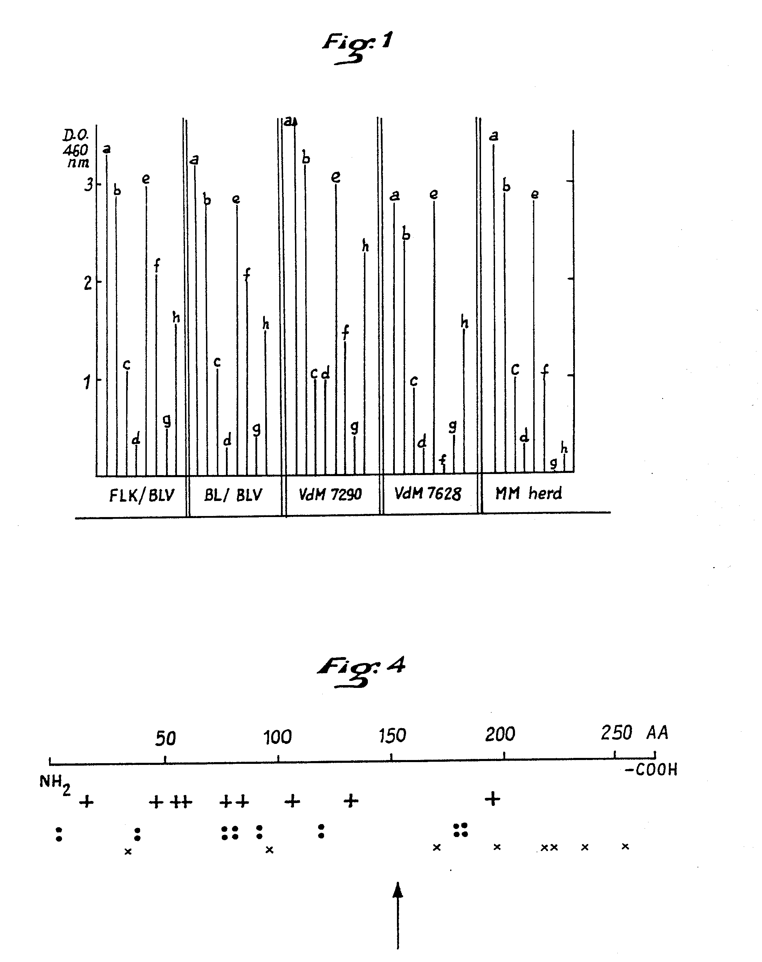

On a illustré sur la figure 1 les résultats d'un test ELISA réalisé avec des particules virales BLV d'origines différentes :

FLK/BLV : un virus de la leucémie bovine BLV cultivé sur des cellules de reins d'agneau foetal ;

BL/BLV : un virus BLV cultivé sur des cellules de poumons de chauves-souris ;

Vd M 7290 et Vd M 7628 : deux isolats de BLV fournis par le Dr. Martin Van der Maaten, Ames, Iowa (USA) ;

MM herd : un isolat de BLV fourni par le Dr. Marc Mammerickx (Belgique).

On a porté en ordonnées la densité optique (D.O.) à 490 nm et chaque barre verticale représente la réactivité des anticorps monoclonaux dirigés contre les épitopes A à H. On voit que les anticorps monoclonaux dirigés contre les épitopes A, B, E présentent une réactivité élevée avec ces épitopes alors que les anticorps monoclonaux dirigés contre les épitopes F, G, H présentent une réactivité plus faible. On observe que les épitopes F sur l'isolat VdM 7628 et G sur l'isolat MM herd sont pratiquement inexistants par suite de mutations intervenues.

## TEST DE FIXATION DES ANTICORPS

Ce test a été effectué dans les conditions suivantes :

- fixation d'une quantité d'antigène gp51 déterminée pour chaque anticorps monoclonal en réalisant différentes dilutions d'antigène ; on choisit la dilution capable de lier 50 % de la radioactivité maximale pouvant être fixée ; cette fixation est effectuée dans des puits d'une plaque de microtitration,

- introduction dans une série de puits des dilutions progressives de 3 en 3 du sérum d'origine bovine à tester (concentration initiale : 1 µl de sérum dans un volume de 50 µl dans le puits) et incubation de la microplaque pendant 5 heures à température ambiante ;

- introduction de 50 µl de solution contenant 10 ng d'anticorps monoclonal marqué radioactivement à l'iode 125 et incubation pendant une nuit à 4°C ;

- lavage des plaques et décrochage de la radioactivité fixée à l'aide d'une solution 2 % dodécylsulfate de Na ;

- comptage de la radioactivité dans un compteur γ ; la radioactivité fixée est exprimée en % de la radioactivité adsorbée en présence d'un sérum bovin normal non compétiteur.

On a illustré sur la figure 2 les résultats obtenus dans des tests de fixation lors desquels les anticorps monoclonaux marqués, dirigés contre les huit épitopes de la gp51, sont mis en compétition avec des quantités décroissantes de sérum bovin infecté (cas de tumeur n° 15). On a porté en abscisses la concentration du sérum (exprimée en µl/ml) et en ordonnées la quantité d'anticorps monoclonaux marqués fixée (exprimée en %).

La courbe avec le symbole H est celle obtenue avec l'anticorps monoclonal spécifique de l'épitope H.

La courbe avec le symbole F est celle obtenue avec l'anticorps monoclonal spécifique de l'épitope F.

La courbe avec le symbole G est celle obtenue avec l'anticorps monoclonal spécifique de l'épitope G.

Les courbes référencées 2-2, 5-5, 6-6, 8-8, 9-9, 10-10 et 15-15 sont les courbes obtenues avec les anticorps monoclonaux respectivement dirigés contre les épitopes B, D, E, C, A, B' et D' de la gp51.

Comme on peut le voir sur cette figure, seuls les anticorps monoclonaux dirigés contre les épitopes F, G et H sont déplacés par l'antisérum bovin.

## TECHNIQUE DE WESTERN BLOT

Cette technique a été mise en oeuvre dans les conditions suivantes :

- suspension du virus BLV obtenue par ultrafiltration et ultracentrifugation,

- dénaturation du virus complet par chauffage à 100°C pendant 5 mn. en présence de dodécylsulfate de sodium (SDS) et d'agent réducteur mercapto-éthanol,

- séparation de protéines virales par électrophorèse en gel de 15 % polyacrylamide, en présence de SDS,

- transfert électrique des protéines ainsi séparées sur membrane de nitrocellulose (tampon d'électrophorèse sans SDS, 2 heures à 4°C sous une intensité de 0,5 A),

- saturation de la membrane avec une protéine inerte, la sérumalbumine bovine, et découpe de la membrane en languettes,

- incubation de chaque languette avec les anticorps monoclonaux à étudier dirigés contre les sites antigéniques de la gp51 et contre la protéine p24 à titre de contrôle (16 h à 20°C),
- après lavage, incubation des languettes avec un sérum de lapin antiimmunoglobulines de souris (2 h à 20°C),
- après lavage, incubation des languettes avec une préparation de protéine A marquée à l'iode 125 (1 h à 20°C),
- après lavage et séchage, autoradiographie des languettes,
- développement photographique et observation de marques correspondant aux protéines virales recherchées.

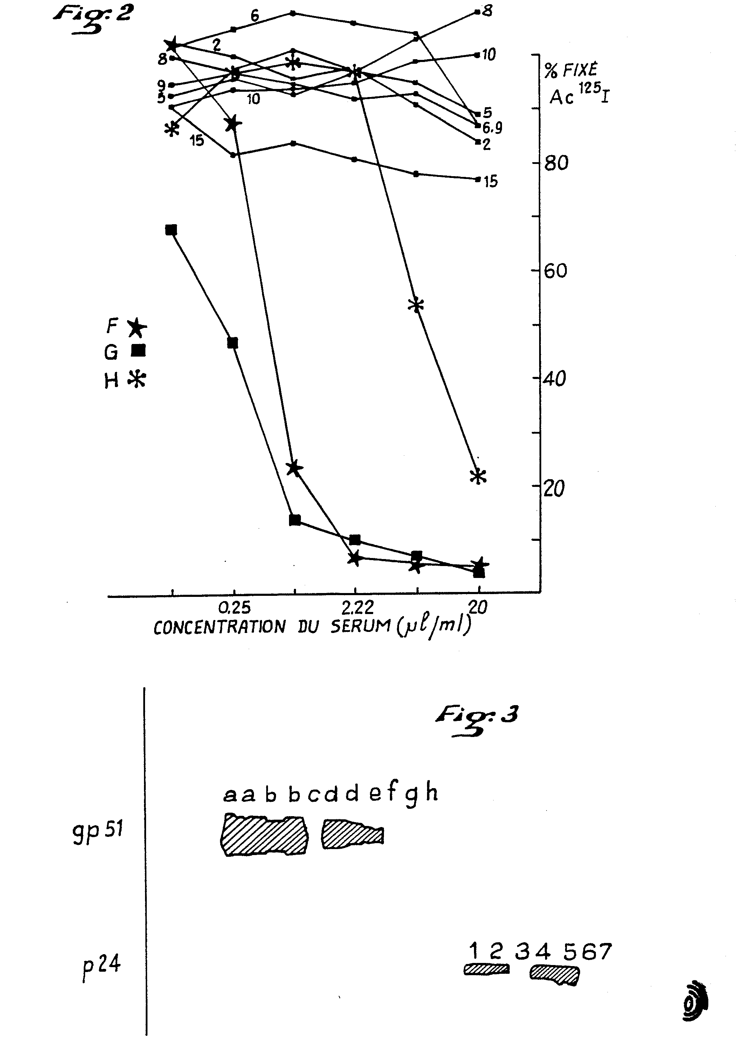

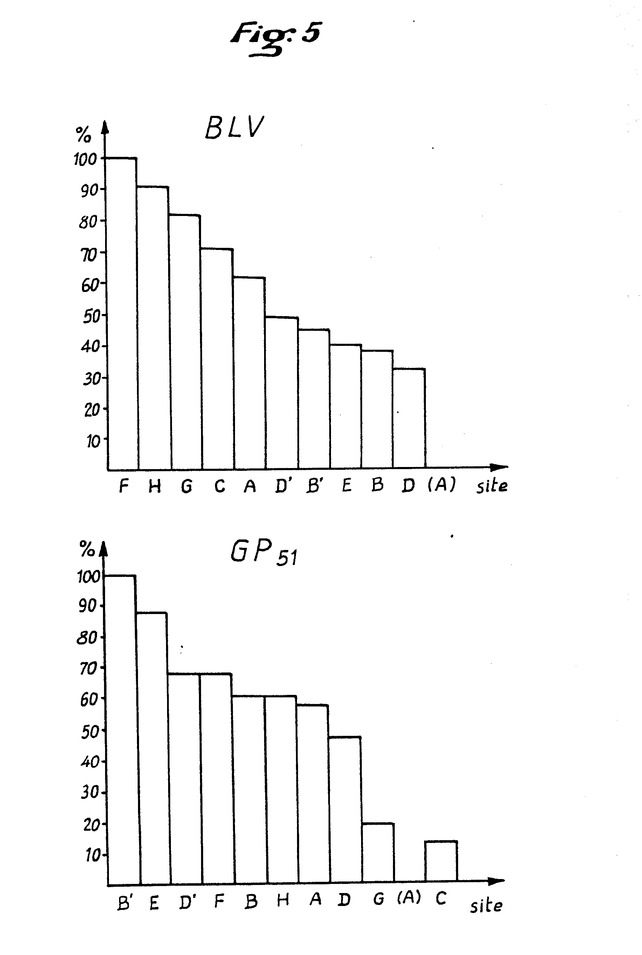

La figure 3 illustre les résultats de l'analyse par Western Blot de particules virales BLV. En marge, sur la gauche de la figure, on a fait figurer les protéines virales repérées. Les bandes indiquées par des le ttres montrent la réactivité de la glycoprotéine gp51 avec les anticorps monoclonaux spécifiques des épitopes A, A', B, B', D, D', E, F, G et H. L'absence de taches colorées sur les bandes C, F, G et H montrent que les épitopes correspondants ont été dénaturés par le traitement initial de l'échantillon. Les bandes repérées par des chiffres illustrent les résultats obtenus avec divers anticorps monoclonaux dirigés contre la protéine p24 du virus BLV, qui est la protéine majeure interne.

On a illustré, sur la figure 5, la réactivité de différents anticorps monoclonaux avec, d'une part, des particules virales entières BLV et avec, d'autre part, une glycoprotéine gp51 purifiée.Les conditions de réaction étaient les suivantes :

- fixation de l'antigène sur les parois de chaque puits d'une plaque de microtitration, à raison de 400 ng de suspension virale purifiée par puits ou de 50 ng d'antigène gp51 purifié par puits,
- après lavage et saturation avec une protéine inerte, la sérumalbumine bovine, introduction dans les puits de 200 µl des dilutions progressives d'anticorps monoclonaux purifiés à tester (1:20 ; 1:60 ; 1:180 ; 1:540 ; 1:1620 ; 1:4860 ; 1:14580 ; 1:43740). Incubation de la réaction pendant 16 h à 4°C,
- après lavage, addition de 10 ng par puits de fragments Fab d'immunoglobulines de chèvre antiimmunogobulines de souris, couplés à l'enzyme peroxydase. Incubation pendant 2 h à 4°C,
- après lavage, révélation de l'activité enzymatique associée aux parois, à l'aide du substrat $H_2O_2$ + O-phénylène diamine,
- après 15 mn, arrêt de la réaction avec HCl 6N et lecture des densités optiques à l'aide d'un spectrophotomètre pour microplaques de titration.

Les résultats sont exprimés pour chaque anticorps en pourcentage de la moyenne des densités optiques aux dilutions 1/20 et 1/60 de l'anticorps monoclonal donnant la densité optique maximale dans le test considéré.

On a fait figurer en abscisses les sites antigéniques reconnus par les différents anticorps monoclonaux.

On voit que les anticorps monoclonaux présentent une grande réactivité avec les particules virales BLV. Les épitopes F, G et H sont probablement les seuls épitopes reconnus sur des particules virales non dégradées. Ces anticorps monoclonaux sont, par contre, faiblement réactifs avec la gp51 purifiée, en particulier le site G.

PREPARATION DU FRAGMENT DE gp51 DE POIDS MOLECULAIRE DE L'ORDRE DE 15 000

On a soumis la gp51 marquée radioactivement à une digestion enzymatique ménagée avec la protéase urokinase.

L'enzyme lyophilisée a été fournie par la firme ABBOTT Laboratories, Ivry-sur-Seine (France). Cette enzyme a été remise en suspension à une concentration finale de 2 mg/ml.

L'antigène purifié gp51 a été marqué radioactivement soit à l'iode 125 par la méthode à la chloramine T de GREENWOOD, F.C. et al (Biochemical Journal (1963), 89, 114-123), soit au tritium sur les résidus lysine de la molécule à l'aide de la technique de méthylation et de réduction au borohydrure de sodium décrite par TACK B.F et WILDER R.L. dans Methods in Enzymology 73, 138-147 (1981) et légèrement modifiée par BRUCK C. et al dans Virology 122, 353-362 (1982)

1 ml de solution contenant 10 ng de iode 125-gp51 ou 11 µg de $^3$H-gp51, on ajoute 50 µl de préparation d'urokinase. Après 45 mn d'incubation à 37°C, on ajoute à nouveau 50 µl de la même préparation d'urokinase, de même qu'après 90 mn d'incubation.

Après un temps total d'incubation de 135 mn, on bloque l'activité de l'urokinase à l'aide d'un inhibiteur de protéase, le phénylméthylsulfonylfluorure PMSF à la concentration de $10^{-4}$M.

Les fragments peptidiques obtenus ont été ensuite immunoprécipités à l'aide des anticorps monoclonaux de la façon qui suit.

Dans un volume final de 200 µl de tampon phosphate isotonique à pH 7,2 contenant 0,2 % de sérumalbumine bovine et 0,2% de Tween 80, on mélange 5 µl de liquide ascitique contenant l'anticorps monoclonal à tester à 25 µl d'échantillon de gp51 digéré par l'urokinase.

Après 20 heures d'incubation à 4°C, on ajoute 100µl de tampon contenant 1 µl de sérum de lapin dirigé contre les immunogobulines de souris et on incube de nouveau 20 heures à 4°C.

On immunoprécipite les complexes antigène-anticorps avec 50 µl d'une préparation de <u>Staphylococcus aureus</u>-protéine A à 10 % pendant 30 mn à 4°C.

La préparation de Staphylococcus aureus-protéine A est recueillie par centrifugation et lavée plusieurs fois avec du tampon phosphate à pH 4 contenant du Triton X-100 0,5 % et du désoxycholate 0,5 %.

La suspension de Staphylococcus aureus-protéine A dans 100 µl de tampon pour l'électrophorèse est ensuite chauffée à 100°C pendant 3 mn et centrifugée pour éliminer les bactéries.

Le surnageant contenant les complexes antigène-anticorps dissociés est recueilli et les protéines sont séparées par électrophorèse en gel de SDS-polyacrylamide 2 %.

Après électrophorèse, le gel est fixé, séché et mis à l'autoradiographie.

Après autoradiographie, les résultats montrent que les anticorps monoclonaux dirigés contre les sites antigéniques A, B, C, D reconnaissent un fragment d'environ 35 000 daltons (fragment I).

Par contre, les anticorps monoclonaux dirigés contre les sites (E), F, G, H reconnaissent un fragment d'environ 15 000 daltons (fragment II).

LOCALISATION DES EPITOPES F, G et H SUR LE FRAGMENT DE POIDS MOLECULAIRE DE L'ORDRE DE 15 000

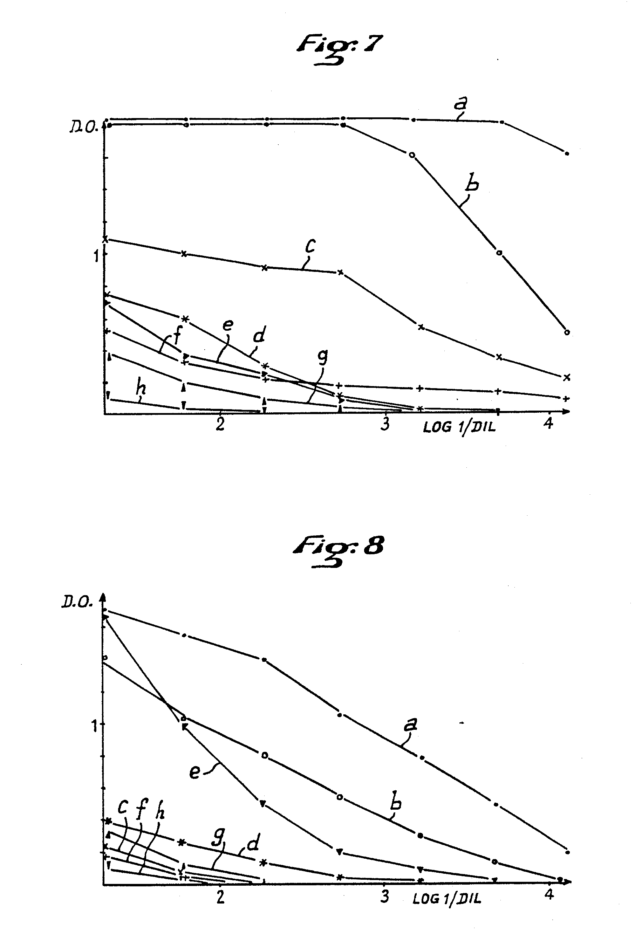

La séquence complète de l'antigène gp51 a pu être facilement déduite de la séquence nucléotidique du gène d'enveloppe codant pour cette glycoprotéine (figure 6).

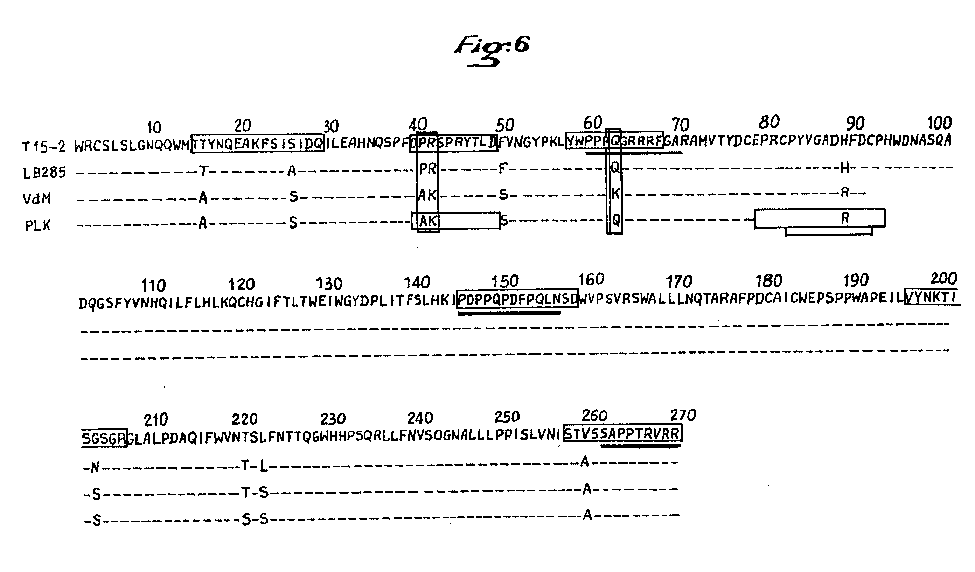

On a représenté sur la figure 4, de façon schématique, la molécule de gp51 avec les sites possibles de glycosylation et l'endroit (symbolisé par la flèche) où s'effectue le clivage de la molécule.

Les symboles utilisés ont la signification qui suit :
- + représente la tyrosine ;
- : représente la cystéine ;
- x représente le site possible de glycosylation.

La cystéine a un rôle important à jouer puisque c'est elle qui permet, par les groupements thiol, la cyclisation du peptide et la structure spatiale des épitopes F, G, H. La tyrosine par son groupement hydroxyle permet le marquage du peptide par un radioisotope iode 125.

Les résultats expérimentaux montrent que :

a) Les sites E, F, G, H, définis par les anticorps monoclonaux sont portés par le fragment II, très peu glycosylé. Or, la séquence en acides aminés de la gp51 montre que la partie NH$_2$ de celle-ci est peu pourvue en sites possibles de glycosylation (deux au lieu de six dans la partie COOH terminale).

b) Après digestion ménagée de l'antigène gp51 marqué à l'iode 125 sur les tyrosines (méthode à la Chloramine T) à l'aide d'une préparation très diluée de protéinase K, les anticorps monoclonaux dirigés contre les sites E, F, G, H précipitent encore plus de 90 % de la radioactivité mise en jeu alors que, dans des conditions identiques, les anticorps monoclonaux dirigés contre les sites A, B, C, D ne précipitent même plus 11 % de la radioactivité. Ces résultats montrent que les épitopes E, F, G, H sont situés dans une région très riche en tyrosine, où se fixe l'iode 125, ce qui est le cas de la partie NH$_2$ terminale de la gp51 (huit tyrosines, pour une seulement dans la partie COOH terminale).

c) L'expérience de Western Blot (figure 3) réalisée en présence de détergent SDS (dodécylsulfate) et d'agent réducteur (mercaptoéthanol) montre la dénaturation des sites antigéniques (C), F, G, H : absence de réactivité avec les anticorps monoclonaux. Par ailleurs, un dosage radioimmunologique de l'antigène gp51 marqué à l'iode 125 permet de mettre en évidence l'absence de réactivité des anticorps monoclonaux dirigés contre les sites (C), F, G, H, si l'antigène à été préalablement traité avec l'agent réducteur mercaptoéthanol, à la dose de 10 mM pendant 15 mn.

Ces résultats font apparaître que la dénaturation des sites C , F, G, H est due avant tout à la présence de l'agent réducteur. Il faut en déduire que ces sites antigéniques possèdent une structure antigénique sous la dépendance de ponts disulfures. Il est à noter que la partie NH$_2$ terminale est très riche en cystéine (six résidus pour seulement deux dans la partie -COOH terminale).

Les résultats énoncés sous a) b) c) montrent que le fragment II portant les épitopes E, F, G, H est la partie NH$_2$ terminale de l'antigène gp51.

Les activités biologiques associées aux antipeptides confirment ces résultats.

PREPARATION DES OLIGOPEPTIDES

On a procédé à la synthèse des peptides qui figurent dans le Tableau I par le procédé en phase solide décrit par MERRIFIELD dans J. Am. Chem. Soc. (1963), 45, 2149-2154.

Dans ce tableau, les lettres L et B indiquent que les peptides proviennent d'origines différentes. Dans la colonne indiquant les séquences d'acides aminés, on a souligné les légères variations pouvant apparaître dans les séquences d'un même peptide (cas du peptide L 39-48). Dans ce même tableau, le crochet en dessous du peptide L78-92 indique la cyclisation du peptide. On a représenté à la figure 6 les séquences d'acides aminés de la glycoprotéine gp51, établies à partir de la séquence d'ADN de quatre variants du virus BLV (T15-2 : cas de tumeur n° 15; LB 285 et VdM : deux isolats de virus et FLK : virus BLV cultivé sur des cellules de rains d'agneau foetal).

Les lettres qui représentent les acides aminés ont les significations suivantes : A Ala, C Cys, D Asp, E Glu, F Phe, G Gly, H His, I Ile, K Lys, L Leu, M Met, N Asn, P Pro, Q Gln, R Arg, S Ser, T Thr, V Val, W Trp, Y Tyr.

Les séquences d'acides aminés qui figurent dans les encadrés ainsi que celles qui ont été soulignées, correspondent aux peptides qui ont été synthétisés par voie chimique.

## SYNTHESE DES OLIGOPEPTIDES.

La synthèse est réalisée selon la technique en phase solide de MERRIFIELD. La chaîne peptidique est préparée à partir du premier acide aminé-C-terminal fixé de manière covalente sur un polymère de styrène et divinylbenzène (pouvant également être de type polyamide ) par une liaison de type ester benzylique (résine chlorométhylée) ou amide (résine benzhydrylamine). Les acides aminés suivants vers la partie N-terminale, sont ajoutés successivement par répétition du cycle d'opérations dont les étapes principales sont :

1) une déprotection du groupe Boc (tertiobutyloxycarbonyle, utilisé pour protéger les fonctions aminées) au moyen d'acide trifluoroacétique (TFA) en solution dans $CH_2Cl_2$ ;

2) une neutralisation des fonctions aminées au moyen de diisopropylamine (DIEA) en solution dans $CH_2Cl_2$;

3) un couplage réalisé par activation des fonctions carboxyliques des acides aminés à introduire au moyen de dicyclohexyl-carbodiimide ou en préparant des esters activés (d'orthonitrophénol, par exemple). Les différents réactifs de couplage sont ajoutés en excès (3 à 6 fois) par rapport à la charge de résine.

En fin de couplage, on vérifie l'absence de fonction amine libre sur la résine au moyen du test à la ninhydrine. En cas de réaction positive, le couplage est répété.

Au cours de la synthèse, les fonctions réactives portées par les chaînes latérales des aminoacides sont protégées au moyen des groupes figurant dans le tableau II.

En fin de synthèse, le peptide est libéré de la résine et de ses groupes protecteurs par traitement durant 1 heure au moyen d'acide fluorhydrique anhydre contenant 10% de p-crésol (V/V) et 5% de diméthylesulfure (V/V).

Seuls les groupes acétamidométhyle (acm) protégeant les cystéines restent intacts à l'issue de ce traitement.

Le peptide est alors purifié par filtration sur gel et chromatographie de partition en phase inversée.

Son identité est contrôlée par analyse d'acides aminés après hydrolyse acide et son homogénéité par chromatographie sur couche de silice dans trois systèmes solvants différents ainsi que par chromatographie liquide pression en phase inverse.

## DEPROTECTION ET CYCLISATION DU PEPTIDE 78-92

20 mg (11,6 μmoles) du peptide 78-92 protégé au niveau des deux résidus cystéines par des groupes S-acétamidométhyle sont dissous dans 1 ml d'une solution aqueuse dégazée d'acide acétique à pH 4.

On ajoute 8 mg (25 μmoles) d'acétate mercurique. La réaction s'effectue à température ambiante pendant trois heures sous agitation et sous azote. On dilue alors le milieu réactionnel par 15 ml d'eau dégazée, et on fait buller de l'$H_2S$ pendant 10 mn sous agitation. La solution est filtrée sur un verre fritté n° 4, puis dégazée par bullage d'azote pendant 2 heures. On dilue alors par 200 ml d'eau dégazée, et on amène à pH 7,5 par de l'ammoniaque diluée. La réoxydation est réalisée par bullage d'air pendant 20 heures. Le milieu réactionnel est alors lyophilisé. Le peptide cyclique obtenu est purifié par filtration sur Biogel P2 ultrafin. Son homogénéité est contrôlée par CCM en différents systèmes solvants et par RP-HPLC par rapport à un témoin non déprotégé.

Son identité est contrôlée par analyse d'acides aminés après hydrolyse acide totale et par mesure du poids moléculaire par F.A.B.S. (Fast Atom Bombardment Spectroscopy).

A partir d'un peptide ainsi obtenu, on peut obtenir un oligomère ayant des propriétés immunogènes renforcées, en procédant comme suit :

5 mg du susdit peptide dissous dans une solution de bicarbonate de sodium 0,1 M sont mélangés avec une solution aqueuse de 25 g de glutaraldéhyde par litre pour obtenir une concentration finale en peptide de 0,1 %. La réaction s'effectue à température ambiante, à l'obscurité et sous agitation ; on laisse la réaction pendant 5 jours. L'oligomère formé peut ensuite être dialysé contre un tampon approprié tel que le PBS.

## COUPLAGE DES OLIGOPEPTIDES AVEC LE SUPPORT

Pour coupler les peptides selon l'invention à une molécule porteuse appropriée telle que keyhole limpet hémocyanine ou la thyroglobuline, en vue de renforcer son immunogénicité, on peut avoir recours à l'une des méthodes décrites ci-après en présence des agents homo-bifonctionnels ou hétéro-bifonctionnels également identifiés ci-après :

I - Agent homo-bifonctionnel tel que la glutaraldéhyde

Les conditions expérimentales peuvent être résumées comme suit.

2,5 mg d'une macromolécule (protéine ou synthétique) dissoute dans 2,5 ml de bicarbonate de sodium 0,1 M sont mélangés à 21 mg de peptide. Après 1 heure de contact sous agitation et à température ambiante, on ajoute en deux étapes de la glutaraldéhyde de manière à obtenir une solution finale à 0,1 % de l'agent couplant. Le contact est maintenu à l'obscurité et à température ambiante pendant 5 jours. On recueille le conjugué formé après dialyse de la solution contre du PBS.

II - Agent hétéro-bifonctionnel liant les fonctions suivantes

1. aminé et carboxyl : - méthode aux anhydrides mixtes :

Les conditions expérimentales suivies correspondent au procédé général décrit par M. L. TILAK dans Tetrahedron Letters 11, 849-854 (1979).

2. Carboxyl et amine ou alcool : - méthode utilisant une carbodiimide hydrosoluble :

Les conditions expérimentales ont été décrites par GOODFRIEND T.L. et al. dans Science 144, 1344 (1964).

3. Amine et sulfhydryl :

De nombreux agents sont utilisés, cependant les plus usuels sont :

a) le 6-maléimido-caproïque-acyl-N-hydroxy-succinimide-ester (MCS). Les procédés de couplage faisant intervenir ce réactif sont décrits par LEE et al. dans Molec. Immunol., 17, 749-756 (1980).

b) le N-succinimidyl-3(2-pyrridyl-dithio)propionate (SPDP).

La méthode employée a été décrite par CARLSSON et al. dans Biochem. J., 173, 723-727 (1978).

IMMUNISATION DES ANIMAUX

Des lapins âgés d'environ trois mois ont été injectés par voie intradermique, à trois reprises et à des intervalles de 15 jours, à l'aide de préparations de conjugués constituées de 1 ml de tampon phosphate isotonique contenant 500 µg de molécules porteuses d'hémocyanine de Keyhole limpet, émulsionné avec un volume égal d'adjuvant complet de Freund. Le sang est prélevé 8 jours après la troisième injection et le sérum est recueilli pour effectuer les différents contrôles.

DETECTION DES ANTICORPS

La détection des anticorps a été réalisée par la méthode ELISA, mise en oeuvre dans les conditions suivantes :

- fixation de l'antigène sur les parois de chaque puits d'une plaque de microtitration, à raison de 400 ng de suspension virale purifiée par puits, ou de 50 ng d'antigène gp51 purifié par puits, ou de 50 ng de peptide de synthèse par puits ;

- après incubation, lavage et saturation avec une protéine inerte, la sérumalbumine bovine, introduction dans les puits de 200 µl des dilutions progressives d'antisérum à tester (dil. 20, 60, 180, 540, 1620, 4860, 14580, 43740). Incubation de la réaction pendant 16 heures à 4°C ;

- après lavage, addition de 100 µl par puits de tampon contenant 10 ng de protéine A couplée à l'enzyme peroxydase si les antisérums utilisés proviennent de lapin, ou addition de 100 µl par puits de tampon contenant 100 ng d'anticorps purifiés par chromatographie d'affinité, dirigés spécifiquement contre les immunoglobulines de l'antisérum utilisé lors des dilutions, et couplés à l'enzyme peroxydase. Incubation pendant 2 heures à 4°C ;

- après lavage, révélation de l'activité enzymatique associée aux parois, à l'aide du substrat $H_2O_2$ + O-phénylènediamine ;

- après 15 minutes, arrêt de la réaction avec HCl 6N et lecture des densités optiques à l'aide d'un spectrophotomètre pour microplaques de titration.

On a étudié par le test ELISA la réactivité des sérums antipeptides contre les peptides de synthèse eux-mêmes et contre les peptides naturels. Les résultats obtenus sont rassemblés dans le tableau III.

A titre de contrôle, on a utilisé un antisérum de lapin contre l'antigène gp51 purifié.

On a représenté sur la figure 7 les courbes de titration des antisérums antipeptides de lapin contre la glycoprotéine gp51 purifiée et sur la figure 8 les courbes de titration des antisérums antipeptides contre les particules virales BLV purifiées, obtenues par la méthode ELISA mise en oeuvre dans les conditions précédemment indiquées. On a porté en abscisses le log de l'inverse de la dilution et en ordonnées la densité optique D.O. mesurée à 460 nm.

Les courbes référencées a sont les courbes de référence obtenues avec l'anti gp51.

Les courbes référencées b sont les courbes obtenues avec l'anti L 255-268.

Les courbes référencées c sont les courbes obtenues avec l'anti B 59-69.

Les courbes référencées d sont les courbes obtenues avec l'anti B 260-268 et l'anti B 144-155.

Les courbes référencées e sont les courbes obtenues avec l'anti L 78-92 linéaire et l'anti L 78-92 cyclique.

Les courbes référencées f sont les courbes obtenues avec l'anti L 144-157.

Les courbes référencées g ou h sont les courbes obtenues avec d'autres antipeptides.

Il ressort de ces résultats que, lorsque l'on utilise la glycoprotéine gp51 purifiée comme antigène, on a dans un ordre décroissant de réactivité

anti-gp51 > anti 255-268 > anti 59-69.

Les autres sérums antipeptides présentent une réactivité beaucoup plus faible.

Lorsque l'on utilise les virions BLV complets comme antigène, l'ordre de réactivité s'établit ainsi :

anti-gp51 > anti 255-268 > anti 78-92 (linéaire ou cyclique).

On a testé les sérums de lapins qui ont été immunisés avec les conjugués pour déterminer leur capacité à neutraliser l'activité biologique du virus BLV en utilisant le test d'inhibition des pseudotypes VSV (BLV) décrit dans ZAVADA J., CERNY L., ZAVADOVA, BOZONOVA J. et ALTSTEIN A.D. dans J. Natl. Cancer Inst. 62, p. 95 à 101 (1979).

Le test d'inhibition de pseudotypes est basé sur des observations de J. ZAVADA et al. montrant que l'infection par le virus de la stomatite vésiculaire (VSV) de cellules chroniquement infectées par le virus BLV fournit des particules virales dont le génome est constitué de celui du virus VSV et dont l'enveloppe est celle du virus BLV (pseudotypes).

Ces pseudotypes VSV/BLV possèdent les propriétés spécifiques liées à la glycoprotéine d'enveloppe gp51 du virus BLV comme la neutralisation et la spécificité d'hôte. Le génome VSV inclus dans ces pseudotypes rend les particules capables de former rapidement (24-36 heures) des plages de lyse sur les cellules de singe qu'on a infecté par ces pseudotypes.

Brièvement, pour la neutralisation des pseudotypes avec les sérums à tester, on mélange 1 ml d'une préparation de pseudotypes pouvant former 200 plages de lyse sur les cellules à tester ($10^{-1}$, $10^{-2}$, $10^{-3}$, $10^{-4}$ et d'autres dilutions intermédiaires). Ces sérums ont été préalablement inactivés à la chaleur (56°C) pendant 30 minutes.

Après une heure d'incubation à 20°C, on inocule 0,5 ml de chaque mélange sur un tapis de cellules de singe se trouvant dans une boîte de Pétri. Après 90 min. d'incubation, l'inoculum est enlevé par lavage avec du tampon isotonique stérile et les cellules sont recouvertes d'une couche d'agar.

Après 24-36 heures d'incubation à 37°C, les cellules sont colorées au rouge neutre et les plages de lyse résiduelles sont comptées.

Le titre en anticorps neutralisants contre le virus BLV est obtenu en déterminant la dilution de sérum avec laquelle on n'obtient seulement que 50 % des plages de lyse obtenues en l'absence de sérum à tester. Les résultats obtenus sont donnés dans le Tableau IV.

Il ressort de ces résultats, que les anticorps dirigés contre le peptide 78-92 présentent une remarquable capacité à inhiber les pseudotypes. On voit de la même façon que les anticorps dirigés conre les peptides 39-48 et 144-157 montrent également une bonne capacité à inhiber les pseudotypes.

On a étudié la réactivité d'un certain nombre de sérums de lapin dirigés contre l'antigène gp51 et de sérums d'animaux infectés par le virus BLV avec les oligopeptides que l'on a synthétisés chimiquement et que l'on a fixés sur les parois d'un puits en matière plastique. Les résultats obtenus sont rassemblés dans le Tableau V. On n'a fait figurer dans ce tableau que les sérums bovins ou ovins donnant un signal de réponse, les autres sérums ne donnant aucune réponse.

Les résultats font apparaître que, parmi les sérums testés, un nombre limité d'entre eux reconnaissent les oligopeptides (sérums bovins n° 15 et 285 et sérums ovins n° 35, 38, 65, 67, 68, 98). L'oligopeptide 78-92 est reconnu à la fois par le sérum bovin (sérum n° 15) et le sérum ovin (sérum n° 65).

Ces résultats permettent, par ailleurs, de faire ces observations :
- on relève de légères différences dans la réactivité des sérums de lapins (sérums n° 167 et 2670), ce qui peut s'expliquer par le fait que les préparations de gp51 administrées n'étaient pas les mêmes et que les animaux receveurs étaient différents ;
- la réactivité d'un sérum de lapin obtenu par inoculation de virions BLV est sensiblement comparable à celle de sérums provenant de lapins auxquels a été administrée la gp51 ;
- les anticorps monoclonaux ne réagissent pas avec les oligopeptides couplés au support.

L'oligopeptide 78-92 présente la remarquable propriété, lorsqu'il est lié à un support protéinique, d'induire des anticorps neutralisants, rendant cet oligopeptide d'un intérêt particulier pour la réalisation de vaccins.

Les propriétés immunogènes de l'oligopeptide 78-92 permettent également son utilisation comme moyen de diagnostic de la présence ou non de virus BLV dans des échantillons sanguins animaux.

Les fragments de glycoprotéine gp51 et les peptides de synthèse, selon l'invention, peuvent être appliqués également en tant qu'antigènes à la recherche d'anticorps, notamment à des fins de diagnostic ou d'analyse ; à la production d'anticorps, y compris d'anticorps monoclonaux, par induction chez des hôtes convenables ; et à la production de vaccins.

| Peptide | Origine du variant | Séquence acides aminés | Lapin immunisé (n°) | Couplage au support protéinique |
|---|---|---|---|---|
| B 260-268 | T15-2 | SAPPTRVR | 2651 | G/THY |
| L 255-268 | T15-2 | STVSSAPPTRVRR | 2664 | G/KLH |
| B 59-69 | T15-2 | PPPQGRRRFGA | 2638 | G/THY |
| L 57-67 | T15-2 | YWPPPQRRRF | 2662 | G/KLH |
| B 144-155 | T15-2 | PDPPQPDFPQLN | 2698 | G/THY |
| L 144-157 | T15-2 | PDPPQPDFPQLNSD | 2658 | G/KLH |
| L 195-205 | T15-2 | VYNKTISGSGP | 2659 | G/KLH |
| L 21-28 | T15-2 | KFSISIDQ | 2649 | G/KLH |
| L 39-48 | T15-2 | CPRSPRYTDL | 2643 | G/KLH |
| L 39-48 | FLK | CAKSPRYTLD | 2657 | G/KLH |
| L 78-92 | FLK | EPRCPYVGADHFDCP | 2653 | G/KLH |
| L 78-92 cyclique | FLK | EPRCPYVGADHFDCP | 2629 | G/KLH |

TABLEAU I

0 284 492

| acide aminé | groupe protecteur |
|---|---|
| Asp | ester benzylique |
| Glu | ester benzylique |
| Ser | ether benzylique |
| Thr | ether benzylique |
| Tyr | 0-2,6 dichlorobenzyle |
| His | tosyle |
| Arg | tosyle |
| Lys | Chlorobenzyloxycarbonyle |
| Cys | S-acetamidométhyle |

TABLEAU II

| antisérum obtenu contre le peptide | Réactivité de l'antisérum contre | | |
|---|---|---|---|
| | peptide | gp 51 purifiée (provenant de cellules FLK) | BLV complet (provenant de cellules FLK) |
| B 260-268 | +++++ | ++ | +/- |
| L 255-268 | +++++ | ++++ | ++++ |
| B 59-69 | +++ | +++ | - |
| L 57-67 | +++ | +/- | - |
| B 144-155 | +++ | ++ | +/- |
| L 144-157 | +++++ | ++ | + |
| L 195-205 | +++++ | +/- | - |
| L 21-28 | +++++ | +/- | - |
| L 39-48 | +++++ | - | - |
| L 39-48 | +++++ | +/- | +/- |
| L 78-92 | +++++ | + | +++ |
| L 78-92 cycl. | +++++ | + | +++ |
| anti gp51 purifiée | / | +++++ | ++++ |

TABLEAU III

| antisérum obtenu contre le Peptide | Origine du Variant du virus | Pouvoir d'Inhibition des pseudo-types |
|---|---|---|
| B 260-268 | 15-2 | - |
| L 255 -268 | 15-2 | - |
| B 59-69 | 15-2 | - |
| L 57-67 | 15-2 | - |
| B 144-155 | 15-2 | - |
| L 144-157 | 15-2 | ++ |
| L 195-205 | 15-2 | - |
| L 21-28 | 15-2 | - |
| L 39-48 | 15-2 | + |
| L 39-48 | FLK | ++ |
| L 78-92 (linéaire) | FLK | ++ |
| L 78-92 (cyclique) | FLK | +++ |
| anti-gp 51 de lapin | FLK | ++++ |

TABLEAU IV

| peptide | Lapin 167 gp51 purifiée | Lapin 2670 gp51 purifiée | Lapin 154 virions BLV | Anticorps monoclonaux (sites A ... H) | Sérums bovins n° | Sérums ovins n° |
|---|---|---|---|---|---|---|
| B 260-268 | +/- | − | + | − | − | 68 (+) |
| L 255-268 | +/- | +++++ | + | − | 15 (+++) | 35,65,68,98, (++++) |
| B 59-69 | +++ | − | +/- | − | 285 (+) | 65,67,68 (+) |
| L 57-67 | − | +/- | +/- | − | − | − |
| B 144-155 | +++++ | +/- | +++++ | − | − | 65,67,68 (+) |
| B 144-157 | +++++ | +/- | +++++ | − | − | 38,65,68,98 (++++) |
| L 195-205 | − | − | + | − | − | − |
| L 21-28 | +/- | − | +/- | − | − | − |
| L 39 48 | +/- | − | +/- | − | − | − |
| L 39-48 | +/- | − | +/- | − | − | − |
| L 78-92 | +++++ | ++ | ++ | − | 15 (+++) | 65 (+++) |
| L 78-92 cycl. | +++++ | +++ | +++ | − | 15 (+) | 65 (+) |

TABLEAU V

0 284 492

**Revendications**

1. Fraction peptidique induisant la formation d'anticorps protecteurs contre le virus de la leucémie bovine (BLV), caractérisée en ce qu'elle comporte une séquence peptidique reproduisant tout ou partie de la séquence du fragment de l'enveloppe glycoprotéinique gp51 du virus de la leucémie bovine portant au moins l'un des épitopes (F, G, H) responsables de l'activité biologique du virus.

2. Fraction peptidique selon la revendication 1, caractérisée en ce qu'elle est constituée par ledit fragment lui-même, ce fragment
- représentant la partie $NH_2$ terminale de la glycoprotéine gp51,
- ayant un poids moléculaire de l'ordre de 15 000 et étant faiblement glycosylé,
- portant en position accessible les épitopes responsables de l'activité biologique du virus.

3. Fraction peptidique selon l'une quelconque des revendications 1 et 2, caractérisée :
- en ce que les épitopes portés par ladite fraction sont reconnus par les anticorps monoclonaux formés contre ceux-ci,
- en ce qu'ils sont reconnus, de façon sélective, par les sérums d'ovins ou de bovins infectés,
- en ce qu'ils sont sensibles à une dénaturation par un agent réducteur.

4. Procédé d'obtention de la fraction peptidique selon l'une quelconque des revendications 2 et 3, caractérisé en ce que l'on soumet la glycoprotéine gp51 à une digestion protéolytique ménagée.

5. Procédé selon la revendication 4, caractérisé en ce que la digestion protéolytique est effectuée par l'urokinase.

6. Procédé selon l'une quelconque des revendications 4 et 5, caractérisé en ce que l'on immunoprécipite les fragments obtenus par clivage protéolytique de la glycoprotéine gp51 par les anticorps monoclonaux produits par des hybridomes préalablement formés et sécrétant lesdits anticorps monoclonaux, ceux-ci reconnaissant le fragment tel que défini dans les revendications 1 à 3, en ce que l'on sépare les complexes protéines-anticorps par électrophorèse sur gel de polyacrylamide et en ce que l'on dissocie lesdits complexes pour obtenir la fraction recherchée.

7. Peptide de synthèse selon la revendication 1, caractérisé en ce qu'il reproduit au moins l'un des épitopes induisant la formation d'anticorps neutralisants, portés par le fragment de gp51 tel que défini dans les revendications 1 à 3.

8. Peptide selon la revendication 7, caractérisé en ce que sa séquence d'acides aminés correspond sensiblement à la séquence 78-92 de la glycoprotéine gp51.

9. Peptide selon la revendication 8, caractérisé en ce qu'il répond à la formule :
Glu - Pro - Arg - Cys - Pro - Tyr - Val - Gly - Ala - Asp-His - Phe - Asp - Cys - Pro
ledit peptide pouvant être protégé ou déprotégé au niveau des cystéines, oligomérisé ou cyclique, la cyclisation s'effectuant par la liaison S-S entre les deux cystéines.

10. Peptide selon la revendication 7, caractérisé en ce que sa séquence d'acides aminés correspond sensiblement à la séquence 144-157 de la glycoprotéine gp51.

11. Peptide selon la revendication 10, caractérisé en ce qu'il répond à la formule
Pro - Asp - Pro - Pro - Gln - Pro - Asp - Phe - Pro - Gln-Leu - Asn - Ser - Asp
ledit peptide pouvant être monomère ou oligomérisé.

12. Peptide selon la revendication 7, caractérisé en ce que sa séquence d'acides aminés correspond sensiblement à la séquence 39-48 de la glycoprotéine gp51.

13. Peptide selon la revendication 12, caractérisé en ce qu'il répond à la formule :
Cys - Pro - Arg - Ser - Pro - Arg - Tyr - Thr - Asp - Leu -
Ledit peptide pouvant être monomère ou oligomérisé.

14. Peptide selon la revendication 12, caractérisé en ce qu'il répond à la formule :
Cys - Ala - Lys - Ser - Pro - Arg - Tyr - Thr - Leu - Aspledit peptide pouvant être monomère ou oligomérisé.

15. Peptide selon l'une quelconque des revendications 7 à 14, caractérisé en ce qu'il est associé à un support macromoléculaire choisi dans le groupe constitué par des protéines naturelles, telles que l'anatoxine tétanique, l'ovalbumine, des sérums albumines, la thyroglobuline, l'hémocyanine ou dans le groupe des supports synthétiques, tels que des polylysines ou des poly(DL-Alanine)poly(L-Lysine).

16. Peptide selon l'une des revendications 7 à 14, caractérisé en ce qu'il est associé à un support protéinique choisi dans le groupe constitué par les protéines virales elles-mêmes.

17. Association de deux au moins des peptides selon l'une quelconque des revendications 7 à 14 par association covalente ou non, entre eux ou sur une protéine porteuse quelconque.

18. Anticorps tels qu'induits in vivo par les fractions peptidiques selon les revendications 1 à 3 et les peptides de synthèse selon les revendications 7 à 14.

19. Anticorps monoclonaux caractérisés en ce qu'ils reconnaissent spécifiquement les épitopes (F, G ou H) portés par les fractions peptidiques selon les revendications 1 à 3, et les peptides de synthèse selon les revendications 7 à 14.

18

20. Vaccin contre la leucémie bovine, caractérisé en ce qu'il associe une fraction peptidique selon l'une des revendications 1 à 3 ou un peptide de synthèse selon l'une des revendications 7 à 14 avec un véhicule ou excipient pharmaceutique du type de ceux utilisés pour la constitution de vaccins.

21. Fraction peptidique selon l'une des revendications 1 à 3, ou peptide de synthèse selon l'une des revendications 7 à 14, caractérisé en ce qu'ils sont utilisés comme réactifs pour détecter la présence du virus BLV.

22. Séquences d'ADN codant pour les peptides selon l'une quelconque des revendications 7 à 14, applicables pour l'expression desdits peptides dans un vecteur d'expression.

23. Application des fractions ou peptides selon l'une quelconque des revendications 1 à 3, 7 à 17 en tant qu'antigènes, à la recherche d'anticorps.

24. Application des fractions ou peptides selon l'une quelconque des revendications 1 à 3, 7 à 17, à l'induction et à la préparation d'anticorps, notamment monoclonaux.

*Fig: 1*

*Fig: 4*

Fig. 2

0284492

% FIXÉ
Ac $^{125}$I

F ★
G ■
H ✻

CONCENTRATION DU SERUM $(\mu\ell/ml)$

Fig. 3

a a b b c d d e f g h

gp 51

p 24

1 2 3 4 5 6 7

**Fig: 5**

# Fig:6

```
        10        20        30        40        50        60        70        80        90       100
T15-2 WRCSLSLGNQQWM|TTYNQEAKFSISIDQ|ILEAHNQSPF[PR|SPRYTL]FVNGYPKL|YWPP[Q]GRRRF]GARAMVTYDCEPRCPYVGADHFDCPHWDNASQA
LB285 ----------------T--------A------------|PR|-----F---------------|Q|----------------------H----------
VdM   -------------A--------S------------|AK|-----S----------------|K|--------------------R--
PLK   --------------A-------S-------------[AK|          |S----------[Q|----------      [              R        ]
```

```
       110       120       130       140       150       160       170       180       190       200
DQGSFYVNHQILFLHLKQCHGIFTLTWEIWGYDPLITFSLHK|PDPPQPDFPQLNSD|WVPSVRSWALLLNQTARAFPDCAICWEPSPPWAPEIL|VYNKTI|

-----------------------------------------------------------------------------------------------------

-----------------------------------------------------------------------------------------------------
```

```
       210       220       230       240       250       260       270
|SGSGR|GLALPDAQIFWVNTSLFNTTQGWHHPSQRLLFNVSQGNALLLPPISLVNI|STVSSAPPTRVRR|
-N---------------T-L--------------------------A----------
-S---------------T-S--------------------------A--------
-S-------------S-S---------------------------A--------
```

0284492

Fig. 7

Fig. 8

Office européen
des brevets

**RAPPORT DE RECHERCHE EUROPEENNE**

Numéro de la demande

EP 88 40 0609

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | BIOLOGICAL ABSTRACTS, vol. 71, no. 3, 1981, page 1920, résumé no. 18339, Biological Abstracts, Inc., Philadelphie, US; D. PORTETELLE et al.: "In animals infected by bovine leukemia virus antibodies to envelope glycoprotein GP51 are directed against the carbohydrate moiety", & VIROLOGY 105(1): 223-233, 1980 * Résumé * --- | 1,4,18, 23,24 | A 61 K 39/21 C 07 K 7/00 C 07 K 1/12 C 07 K 17/00 A 61 K 39/42 C 12 N 15/00 |
| X,D | CHEMICAL ABSTRACTS, vol. 103, 1985, page 429, résumé no. 52362j, Columbus, Ohio, US; D. PORTETELLE et al.: "Use of monoclonal antibodies in the study of BLV glycoprotein GP51. Consequences for a vaccine", & COMM. EUR. COMMUNITIES [REP] EUR 1984, EUR 8471, Agriculture 45-51 * Résumé * --- | 1-3,19, 20,24 | |
| X,D | BIOLOGICAL ABSTRACTS, vol. 76, no. 4, 1983, page 2842, résumé no. 26246, Biological Abstracts, Inc., Philadelphie, US; C. BRUCK et al.: "Monoclonal antibodies define 8 independent antigenic regions on the bovine leukemia virus envelope glycoprotein GP51", & VIROLOGY 122(2): 342-352, 1982 * Résumé * --- | 1,19,24 | **DOMAINES TECHNIQUES RECHERCHES (Int. Cl.4)** <br><br> A 61 K |
| X | EP-A-0 051 216 (THE REGENTS OF THE UNIVERSITY OF CALIFORNIA) * Page 2, ligne 31 - page 3, ligne 8; page 6, lignes 15-36; page 11, exemple III * --- -/- | 1-4,20 | |

Le présent rapport a été établi pour toutes les revendications

| Lieu de la recherche | Date d'achèvement de la recherche | Examinateur |
|---|---|---|
| LA HAYE | 26-05-1988 | REMPP G.L.E. |

EPO FORM 1503 03.82 (P0402)

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | BIOLOGICAL ABSTRACTS/RRM, vol. 30, 1986, résumé no. 30096631; D. PORTETELLE et al.: "Importance of the envelope glycoprotein GP-51 in the diagnosis of bovine leukemia virus infection and in the development of an anti-bovine leukemia virus subunit vaccine", & J. CELL. BIOCHEM. SUPPL. 1986, vol. 0, no. 10, part A, p. 209 * Résumé * ----- | 1-24 | |
| | | | **DOMAINES TECHNIQUES RECHERCHES (Int. Cl.4)** |

Le présent rapport a été établi pour toutes les revendications

| Lieu de la recherche | Date d'achèvement de la recherche | Examinateur |
|---|---|---|
| LA HAYE | 26-05-1988 | REMPP G.L.E. |

EPO FORM 1503 03.82 (P0402)